# EUROPEAN PATENT SPECIFICATION

(11) **EP 2 844 151 B1**
(45) Date of publication and mention of the grant of the patent: **14.06.2017**
(21) Application number: 13722839.1
(22) Date of filing: 26.03.2013
(51) Int. Cl.: A61B 17/00, B05C 17/005

(54) **DEVICE FOR APPLYING ADHESIVE FROM A VIAL OR AMPOULE WITH DEFORMABLE WALLS AND AN ADHESIVE DISPENSING KIT THAT INCLUDES SAID APPLICATOR DEVICE**
VORRICHTUNG ZUM AUFTRAGEN EINES HAFTSTOFFS AUS EINER PHIOLE ODER AMPULLE MIT VERFORMBAREN WÄNDEN UND HAFTSTOFFABGABEKIT MIT DER BESAGTEN AUFTRAGSVORRICHTUNG
DISPOSITIF POUR APPLIQUER UN ADHÉSIF DEPUIS UN FLACON OU UNE AMPOULE DOTÉ(E) DE PAROIS DÉFORMABLES ET KIT DE DISTRIBUTION D'ADHÉSIF COMPRENANT LEDIT DISPOSITIF APPLICATEUR

(30) Priority: 27.03.2012 ES 201230454
(43) Date of publication of application: 11.03.2015
(73) Proprietor: B. Braun Surgical, S. A., 08191 Rubi (ES)
(72) Inventor: TURÓN DOLS, Pau, 08191 Rubí (ES); DEL CASTILLO RIESTRA, Luis Felipe, 08191 Rubí (ES); IBÁÑEZ GARCÍA, Nuria, 08191 Rubí (ES); SÁNCHEZ GARRIDO, Ricardo, 08191 Rubí (ES); PABLO, Miguel, 08191 Rubí (ES); GALINDO ANGUERA, Magí, 08191 Rubí (ES); PARÍS CASADELLÀ, Ferran Alex, 08191 Rubí (ES)
(74) Representative: Oficina Ponti, SLP
(86) International application number: PCT/IB2013/052413
(87) International publication number: WO 2013/144849

(56) References cited:
- EP-A1- 1 454 844
- WO-A2-2008/001067
- US-A- 4 692 157

## Description

The present invention relates to a device for dispensing and applying with precision adhesive from a vial or ampoule with deformable walls and an adhesive dispensing kit that includes said applicator device.

In particular, the present invention refers to a device that can apply with precision an adhesive for medical use in cases such as the joining of biological tissue for closing wounds, the fixing of implants in surgical repair treatments, as a sealant of blood vessels and viscera to prevent seepage of the fluids they transport, or as a hemostatic.

### Background of the invention

The principal use of medical adhesives for closing wounds is well-known. These adhesives are normally applied in the form of a fine continuous film over the edges of the wound previously brought together. The same adhesives can be used for fixing meshes in surgical treatments for hernia repair. In this case, the adhesive is applied by depositing a drop between the structure of the mesh and the biological tissue to which it must be fixed.

Medical adhesives are normally low-viscosity liquids with a high affinity for the tissue and which wet it very easily. Some of these adhesives are commercialised in vials or ampoules made of plastic material with walls that can be deformed to allow dispensing of the adhesive. The product of the Histoacryl® brand of the B.Braun group is one example of this type of adhesive commercialised in plastic vials or ampoules.

The plastic material that constitute the ampoules or vials of the Histoacryl® brand have an appropriate thickness and density to help guarantee the product's stability and provide the walls of the container with the appropriate degree of flexibility and module to facilitate the controlled application of the film or drop by drop application of the adhesive through the discharge mouth of the container itself.

The plastic vials or ampoules such as those of the Histoacryl® brand are very suitable for external application with easy access on the part of the user. However, these same vials or ampoules are not very suitable for use in areas of difficult access in open surgery processes, given that the discharge mouth of the vial or ampoule does not have either the length, or the rigidity, or the spatial shape necessary for reaching the internal anatomical structures on which the adhesive needs to be applied, such as for example, in the fixing of a mesh during hernia repair surgery.

The state of the art is familiar with devices for applying medical adhesives, which present the advantage of facilitating the user's application of the adhesive.

Patent documents EP1633255, EP2269517 and EP1799609 describe some of these devices which present the particular feature of being based on breaking the glass walls of a vial or ampoule that is disposed in a housing having flexible walls. Once the vial or ampoule is broken, the compression of the flexible walls of the housing enables dispensing of the adhesive through a filter and a cannula. The device of patent document EP1799609 is especially appropriate for use in areas of difficult access in open surgery operations.

Patent document WO2008/001067 discloses a device for applying liquid adhesive for medical use that comprises a receiver to accommodate a sealed ampoule of surgical adhesive. The ampoule is formed of a frangible material, which when fractured, releases the surgical adhesive. The applicator further comprises a blunt cylindrical body of a surgical adhesive-permeable foam inserted into an open end of the receiver.

According to patent document WO2008/001067, the device includes a pair of wings with pressure barbs for applying a compressing force to the walls of the receiver to fracture the ampoule accommodated within this receiver. Once the ampoule has been fractured, the expressed adhesive passes through the foam before arriving to the tip of the pipette.

The devices of the aforesaid patents present the disadvantage of having to use filters to avoid exposing the patient to pieces of broken glass from the vial or ampoule. Another disadvantage presented by said devices lies in the fact that they are one-use only, since once the glass ampoule or vial has been broken and the adhesive supplied, the device is unusable for a new use.

### Description of the invention

The object of the present invention is to resolve the disadvantages of the devices in the state of the art through the development of a device for applying adhesive and an adhesive dispensing kit that present the advantages described hereafter.

Pursuant to this objective, according to a first aspect, the present invention provides a device for applying a medical liquid adhesive in open surgery operations from a vial or ampoule as claimed in claim 1.

Pursuant to the same objective, according to a second aspect, the present invention provides a medical adhesive dispensing kit as claimed in claim 10. Said kit comprises a vial or ampoule that contains liquid adhesive for medical use for application in the joining of biological human or animal tissues, in the fixing of implants or as a hemostatic.

The device of the present invention presents the advantage of it being possible to introduce and extract the vial or ampoule through an aperture, and to couple or decouple it from the joining portion of the cannula, using the coupling means that the cannula is provided with. Thanks to this, the same device can be used with a plurality of vials or ampoules, which translates into a very significant financial saving in respect of other devices in the state of the art which are one-use only.

Another advantage of the present invention lies in the fact that it provides a sterile applicator device capable of being used with vials or ampoules having deformable walls, such as the vials or ampoules of the Histoacryl® brand of the B.Braun group.

Thanks to this device, said vials or ampoules with deformable walls, which up until now were only used for external applications, can be used in internal surgery processes since the cannula of the device is much better suited than the discharge mouth of the vial or ampoule to allow greater depth of access to the internal anatomical structures through the performed incision.

According to a preferred embodiment, said aperture for the insertion of the vial or ampoule is provided at one end of said body, the cannula of the device including a joining portion that can be coupled in a removably manner to said open end, said vial or ampoule being capable of being extracted through said open end together with the cannula, in order to carry out a replacement.

This embodiment presents the advantage that the open end is configured such that the vial can be extracted in a very convenient way upon removing the cannula, once use of the device has ended, allowing both the cannula and the vial to be replaced in a single operation.

Also, this embodiment makes it possible to provide a dispensing kit with a spare cannula that can be coupled to said body and two vials or ampoules of adhesive, in such a way that both the cannula and the vial can be replaced in the event of becoming unusable.

As claimed, the actuator means comprise a pair of actuator elements opposite each other with relative movement in respect of said body, said actuator elements being capable of exerting pressure on two opposite walls of said vial or ampoule, each of said actuator elements comprising a compression tooth of said vial or ampoule, the compression teeth of said actuator elements determining two pressure points opposite each other through which deformation of the walls of said vial is carried out.

It has been observed that the pressure exerted locally on the aforesaid pressure points, due to their limited section, facilitates the pumping of the adhesive from the vial or ampoule as less effort is required to achieve the pressure of expulsion, guaranteeing in turn the supply of accurate doses of adhesive, which is especially appropriate when working with vials or ampoules that contain liquid adhesive for medical use.

Likewise, as claimed, the actuator means comprise a tightening pincer that is mounted on a support inside said body, the arms of said pincer configuring said pair of actuator elements, said support acting as a pivot support for said arms, the body of said device being provided with a pair of apertures through which the arms of said pincer emerge to be actuated, a portion of each one of said arms being disposed in correspondence with the cavity for housing said vial or ampoule.

This configuration is very practical to manufacture, at the same time as it provides an ergonomic design of the body which facilitates the manipulation of the device, in addition to the extraction and insertion of the vial or ampoule from the aforesaid housing cavity.

Advantageously, the internal edge of the portion of each one of the arms of the tightening pincer comprises one of said compression teeth and an element that acts as a stop for said vial or ampoule, said elements that act as a stop facilitating the securing of said vial or ampoule when said compression teeth deform the walls of said vial or ampoule.

This detail once again contributes to ensuring the precise supply of the amount of liquid adhesive, given that while compression is carried out to pump the adhesive using the aforesaid teeth, said elements that act as a stop preventing the movement of the vial or ampoule.

Again advantageously, the housing cavity of said body comprises a support element for the base of said vial or ampoule and lateral guide tracks to guide the position of said vial or ampoule.

These details of the housing cavity help to fix the position of the vial or ampoule, facilitating control of the pumping of the adhesive.

According to the same preferred embodiment, the joining portion of the cannula comprises a wall for insertion in the opening of the end of said body, said insertion wall and the wall that defines the aperture of said end including mutual engaging means for safe coupling.

Advantageously, the wall that defines the aperture of said end includes a longitudinal groove for the insertion of one projection of the cannula, the same wall of said end including a transversal groove associated to said longitudinal groove to allow the engaging of said projection upon turning the cannula in respect of said end.

It is important to guarantee safe coupling of the cannula to the body of the device, given that, as already mentioned, the device is especially indicated for allowing access to internal anatomical structures in internal surgical processes where minimum risk must be guaranteed.

Advantageously, the coupling means of the discharge mouth of said vial or ampoule comprise a cavity in the joining portion of the cannula sized to allow adjustment of the discharge mouth of said vial or ampoule.

In this way, it is very easy to couple and decouple the vial or ampoule in the cannula.

Again advantageously, the body of said device comprises two detachable body portions to allow access to the housing cavity of the vial.

Preferably, the free end of the cannula comprises a bend that configures a curve.

The curved shape of the tip facilitates manoeuvrability between the different structures by increasing the angle of coverage of adhesive application due to its curved tip.
Again preferably, the tip of the cannula presents a section of a lower size so that it can be cut.
In this way, if the tip of the cannula becomes blocked with adhesive during use, said tip can be cut so as to continue dispensing the adhesive.
Advantageously, said body comprises a side window situated in correspondence with the housing cavity of said vial or ampoule, said window making it possible to see the level of adhesive of the vial or ampoule.

In the present disclosure, cannula shall be understood to mean, preferably, a tube with a length of equal to or more than 5 cm to guide the discharge of adhesive from the vial or ampoule. Again preferably, said tube will be sterile and appropriate for supplying adhesive in surgery operations. Advantageously, said cannula will be transparent to carry out better dispensing.

In the present disclosure, a vial or ampoule with deformable walls shall be understood to mean, preferably, a vial or ampoule provided with walls whose material, advantageously plastic, possess a degree of flexibility and module suitable for facilitating the controlled application of the adhesive, preferably, in the form of a film or drop by drop, through the discharge mouth of said vial or ampoule, by exerting a force of pressure on said vial or ampoule.

### Brief description of the drawings

For a better understanding of what has been described a set of drawings is attached which, schematically and only by way of a non-limiting example, represent a practical case of embodiment.

In said drawings,
figure 1 shows a view in perspective of an embodiment of the device of the present invention in a position of use.
figure 2 shows a side view of a portion of the body of the device of figure 1 and of the tightening pincer that acts on the walls of the vial or ampoule.
figure 3 shows a longitudinal section of the cannula of the device of figure 1.
figure 4 shows a view in perspective of the inside of the device of figure 1 in which the position of the vial inside the housing cavity can be appreciated.
figures 5 to 8 show a plurality of schematic views representing the sequence of steps to insert and extract the vial or ampoule from the device.
figure 9 represents an adhesive dispensing kit that includes the device of figure 1, two cannulas and two vials or ampoules of adhesive.

### Description of a preferred embodiment

Next is a description of a preferred embodiment of the device for applying adhesive of the present invention with reference to the attached drawings.

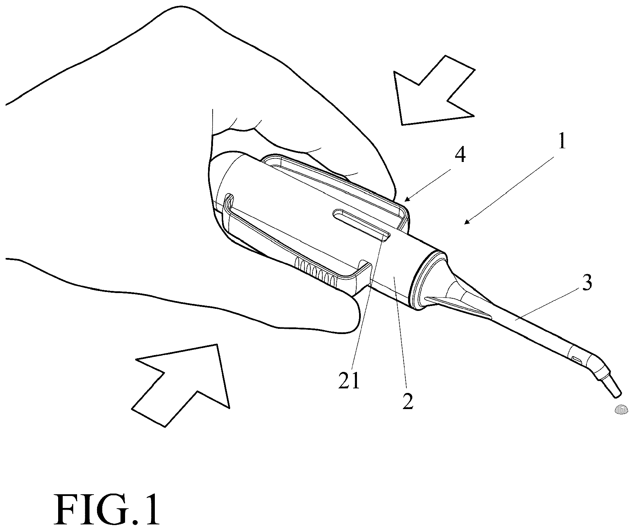

Figure 1 shows an external view in perspective of an embodiment of the device 1 which comprises a body 2 with a substantially cylindrical shape that is provided with a housing cavity for a vial or ampoule with deformable walls, a cannula 3 or tube of approximately 7 cm to guide the discharge of adhesive from said vial or ampoule, and actuator means on the walls of said vial or ampoule that are configured by a tightening pincer 4.

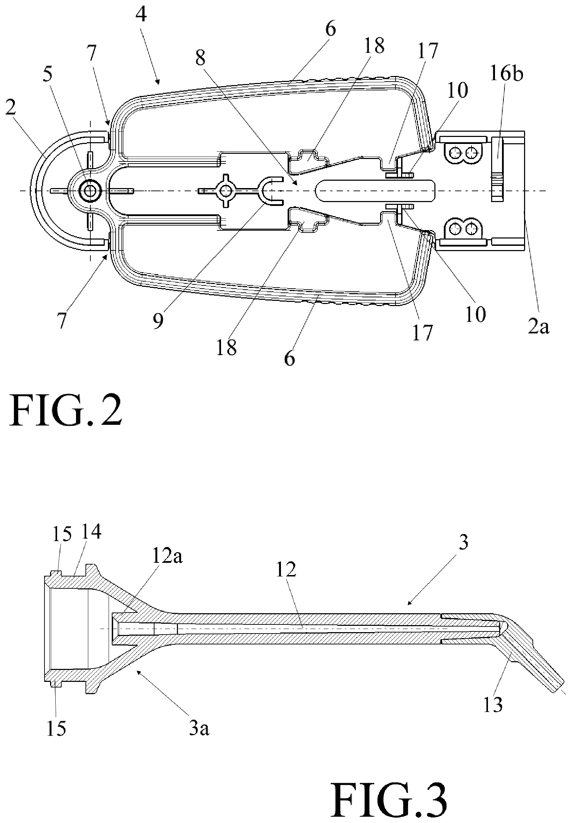

Figure 2 shows a side view of the aforesaid pincer 4 mounted on a support 5 of the inside of the body 2. This pincer 4 presents two opposite arms 6 which act as actuator elements of the walls of the vial or ampoule. These arms 6 emerge from a pair of apertures 7 of the body so that they can be actuated by the user. The arms 6 can move in respect of the support 5, which acts as a pivot, to exert a force of compression on the walls of the vial or ampoule and dispense the adhesive.

In the same figure 2 the housing cavity 8 of the vial or ampoule can be appreciated inside the body 2. This cavity 8 includes a supporting element 9 for the base of the vial or ampoule, in addition to a pair of lateral guide tracks 10 to guide the position of said vial or ampoule.

In the described embodiment, the body 2 is made up of two detachable portions, which define at their ends 2a an aperture 11 through which the vial or ampoule can be inserted and extracted. In the aforesaid aperture 11 a joining portion 3a of the cannula 3 is coupled in a detachable manner which guides the discharge of adhesive. Figure 3 shows a section of said joining portion 3a of the cannula 3 and of a conduit 12 of the same cannula 3 for evacuation of the adhesive.

As shown in figure 3, the section of conduit 12 gradually decreases towards the tip of the cannula 3 to facilitate cutting of the cannula 3 in the event of blockage during use. The free end of the conduit 12 comprises a bend 13 that configures a curve in the tip of the cannula 3 to facilitate manoeuvrability of the device 1, while the opposite end of the same conduit 12 comprises an extension that defines a cavity 12a inside portion 3a to allow adjustment of the discharge mouth of the vial or ampoule.

To ensure safe coupling of the cannula 3 in the body 2, joining portion 3a of the cannula 3 presents an insertion wall 14 with a pair of projections 15 to engage in grooves 16a, 16b of the walls that define the aperture 11 of the body 2. In figure 2 one of said grooves 16b can be appreciated which runs transversally in the wall of a portion of the body 2 to allow engaging of the projection 15 when the cannula 3 is turned in respect of said body 2.

In reference again to the pincer 4 shown in figure 2, it can be seen that a portion of the arms 6 of said pincer 4 is disposed in correspondence with the housing cavity 8 of the vial or ampoule. The internal edge of the portion of each one of the arms 6 includes a compression tooth 17 and an element 18 that acts as a stop for the vial or ampoule.

As mentioned in the description of the invention, the compression teeth 17 of the arms 6 determine two opposite pressure points through which deformation of the walls of the vial or ampoule is achieved, while the elements 18 that act as a stop determine two securing points that prevent the movement of the vial or ampoule while compression is carried out for pumping of the adhesive. Thanks to this, the supply of liquid is carried out in a very precise manner.

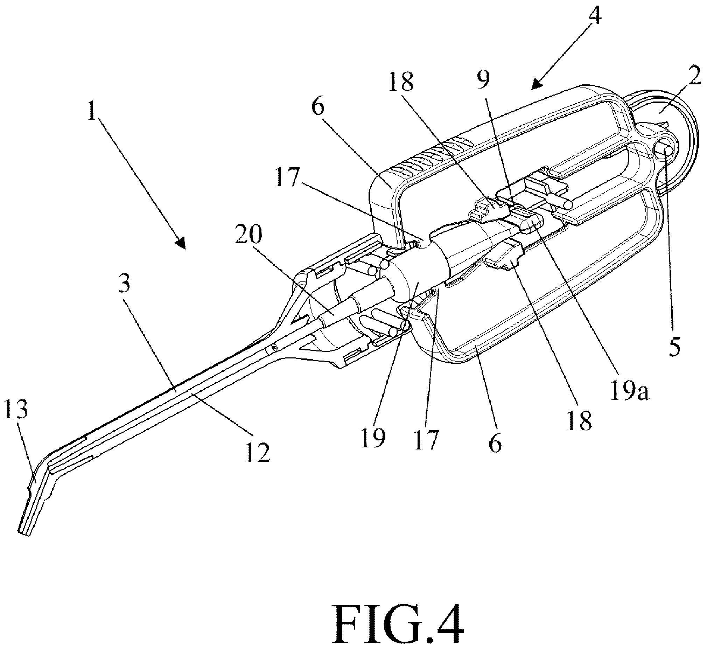

Figure 4 shows a view in perspective of the inside of the device 1 with a vial or ampoule 19 of adhesive disposed in the position of use. In the embodiment described, the vial or ampoule 19 contains liquid adhesive for medical use, preferably, a polymerisable adhesive, for application in the joining of biological human or animal tissues, in the fixing of implants or as a hemostatic. Specifically, it is a vial or ampoule 19 of adhesive commercialised with the Histoacryl® brand of the B.Braun group. The walls of this vial or ampoule 19 are made of plastic and have an appropriate thickness and density to help ensure the stability of the product and to provide the container with a degree of flexibility and module that facilitates the controlled application of the adhesive.

As can be seen in the aforesaid figure 4, the vial or ampoule 19 has a discharge mouth 20 which is coupled to the inside of the cavity 12a of the joining portion 3a of the cannula, and a sealing base 19a which is supported on the supporting element 9 of the housing cavity 8.

In the position of use shown in figure 4, the vial or ampoule 19 is positioned between the arms 6 of the pincer 4, ready for being pressed by the compression teeth 17 of said arms 6 while the elements 18 that act as a stop retain in position said vial or ampoule 19.

Next is a description of the functioning or mode of use of the claimed device 1 with reference to drawings 5 to 8.

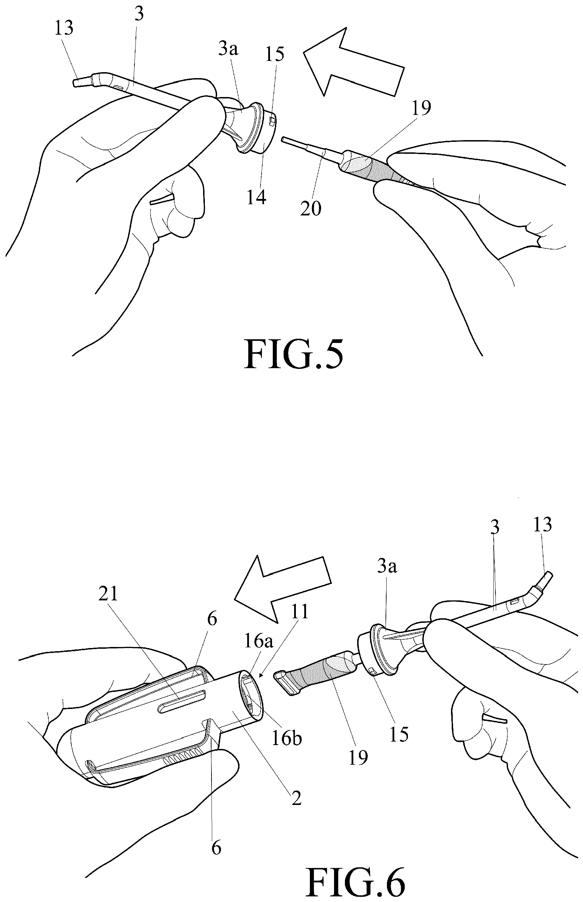

In the first instance, the discharge mouth 20 of the vial or ampoule 19 is opened and said discharge mouth 20 is inserted in the cavity 12a of the joining portion 3a of the cannula 3 (see figure 5).

Once the vial or ampoule 19 is secured in the cannula 3, this vial or ampoule 19 is introduced inside the body 2 of the device 1 through the aperture 11 provided in the end 2a of the same body 2 (see figure 6).

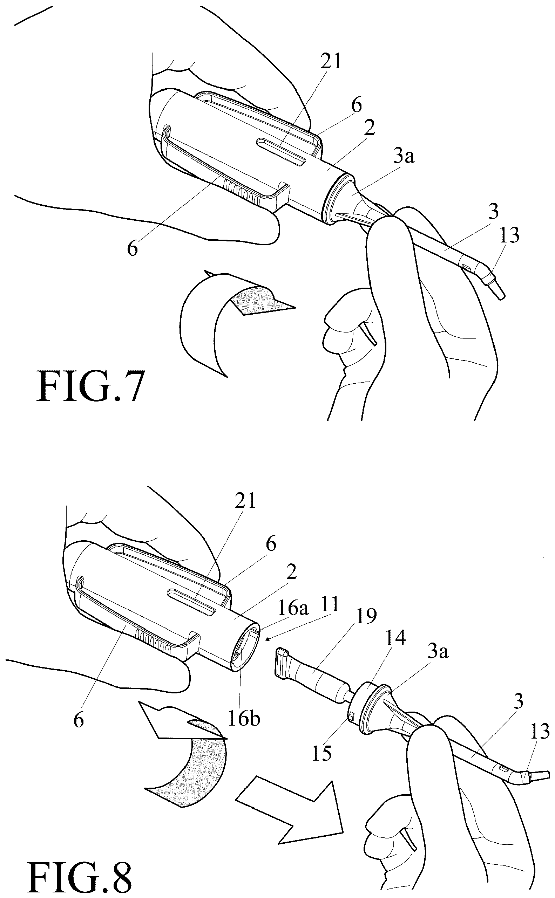

As previously mentioned, the joining portion 3a of the cannula 3 is coupled to the aperture 11 by means of an insertion wall 14 that has a pair of projections 15 to engage in the grooves 16a,16b provided in the walls of the end 2a of the body 2. In figure 6 a first longitudinal groove 16a can be seen which allows the insertion of a projection 15, and a second transversal groove 16b which allows the engaging of the same projection 15 when the cannula 3 is turned approximately 90° clockwise, as shown in figure 7.

Once the joining section 3a of the cannula 3 is safely coupled, the device 1 is ready for use, and the adhesive can be supplied by exerting a slight pressure on the opposite walls of the vial or ampoule 19, by means of the compression teeth 17 of the arms 6.

In the described embodiment, to appreciate the level of adhesive in the vial or ampoule 19, a window 21 has been provided in the body 2.

Once the content of the vial or ampoule 19 has finished, said vial or ampoule 19 is extracted through the aperture 11 together with the cannula 3 in order to carry out a replacement. To this effect, the cannula 3 is made to turn in an anti-clockwise direction until the projections 15 of the insertion wall 14 of said cannula 3 engage in the longitudinal groove 16a of the end 2a of the body 2, and the cannula 3 can be extracted (see figure 8).

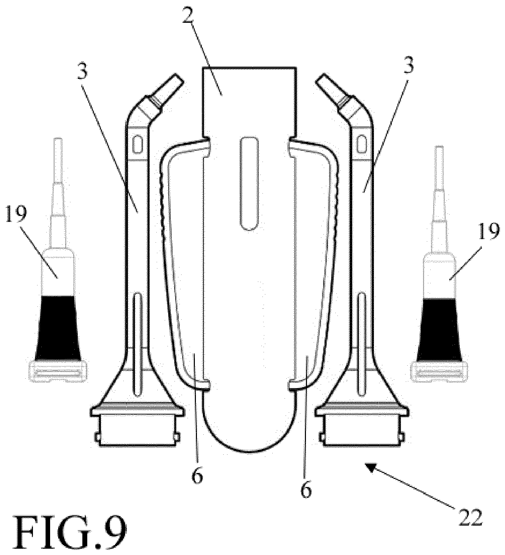

The device 1 of the present invention will be supplied preferably in the form of a sterile dispensing kit 22 with two cannulas 3 and two vials or ampoules 19 of adhesive in order to perform several uses during the same surgical process (see figure 9).

Despite the fact that reference has been made to a specific embodiment of the invention, it is obvious to a person skilled in the art that the described device 1 is capable of numerous variations and modifications, and that all mentioned details can be replaced by others which are technically equivalent, without leaving the scope of protection defined by the attached claims. For example, although in the present embodiment the device 1 comprises a body 2 with an aperture 11 at one end 2a, similar results could be obtained if the device 1 had the aperture for extraction and insertion of the vial or ampoule 19 in a side of the body 2. Similarly, although in the present embodiment reference has been made to the use of a vial or ampoule 19 with deformable walls of the Histoacryl® brand, similar results could be obtained with another type of vial or ampoule having likewise deformable walls with a degree of flexibility and module that facilitated the controlled application of the film or drop by drop application of the adhesive for medical use, and a discharge mouth 20 capable of adapting to the cannula 3.

## Claims

1. A device (1) for applying a medical liquid adhesive in open surgery operations from a vial or ampoule (19) with deformable and flexible walls, the device comprising a body (2) provided with a housing cavity (8) of said vial or ampoule (19), a cannula (3) for guiding the discharge of adhesive from said vial or ampoule (19) and means (4,5,6,17,18) for actuating the walls of said vial or ampoule (19), said actuator means (4,5,6,17,18) being capable of exerting a compression force on the walls of said vial or ampoule (19) to dispense said adhesive, wherein
- said actuator means comprise a tightening pincer (4) that is mounted on a support (5) inside said body (2), the arms of said pincer (4) configuring a pair of actuator elements (6) opposite each other with relative movement in respect of said body (2), said support (5) acting as a pivot for said arms (6) and the body (2) of said device (1) being provided with a pair of apertures (7) through which the arms (6) of said pincer (4) emerge to be actuated, a portion of each one of said arms (6) being disposed in correspondence with the housing cavity (8) of said vial or ampoule (19) and each one of said arms (6) including a compression tooth (17) of said vial or ampoule (19), said compression teeth (17) determining two opposite pressure points through which deformation of flexible walls of said vial or ampoule (19) is achieved to pump said adhesive from said vial or ampoule (19) through the cannula (3), wherein
- the body (2) of said device (1) comprises an aperture (11) which is configured to allow the insertion of said vial or ampoule (19) in the aforesaid housing cavity (8), said cannula (3) including a joining portion (3a) associated to said body (2), said joining portion (3a) of the cannula (3) being provided with means (12a) for coupling a discharge mouth (20) of said vial or ampoule (19), it being possible to decouple said vial or ampoule (19) from said cannula (3) and to extract it through the aforesaid aperture (11) in order to carry out a replacement once the liquid of said vial or ampoule (19) has been dispensed.

2. Device according to claim 1, wherein said aperture (11) for the insertion of the vial or ampoule (19) is provided in one end (2a) of said body (2), the cannula (3) of the device including a joining portion (3a) that can be coupled in a detachable manner to said open end (2a), it being possible to extract said vial or ampoule (19) through said open end (2a) together with the cannula (3) in order to carry out a replacement.

3. Device according to claim 1, wherein the internal edge of the portion of each one of the said arms (6) comprises one of said compression teeth (17) and an element (18) that acts as a stop for said vial or ampoule (19), said elements (18) that act as a stop facilitating the securing of said vial or ampoule (19) when said compression teeth (17) deform the walls of said vial or ampoule (19).

4. Device according to any of the preceding claims, wherein the housing cavity (8) of said body comprises a supporting element (9) for the base (19a) of said vial or ampoule (19) and lateral guide tracks (10) to guide the position of said vial or ampoule (19).

5. Device according to any of claims 2 to 4, wherein the joining portion (3a) of the cannula (3) comprises an insertion wall (14) in the aperture (11) of the end (2a) of said body (2), said insertion wall (14) and the wall that defines the aperture (11) of said end (2a) including mutual engagement means (15,16a,16b) for safe coupling.

6. Device according to any of the preceding claims, wherein the coupling means of the discharge mouth (20) of said vial or ampoule (19) comprise a cavity (12a) sized to allow adjustment of the discharge mouth (20) of said vial or ampoule (19).

7. Device according to any of the preceding claims, wherein the free end of the cannula comprises a bend that configures a curve.

8. Device according to any of the preceding claims, wherein the tip of the cannula (3) presents a section of a lesser size so that it can be cut.

9. Device according to any of the preceding claims, wherein said body (2) comprises a side window (21) situated in correspondence with the housing cavity (8) of said vial or ampoule (19), said window (21) making it possible to see the level of adhesive of the vial or ampoule (19).

10. Medical adhesive dispenser kit (22) comprising a device (1) according to any of claims 1 to 9, and a vial or ampoule (19) that contains a liquid adhesive for medical use, said vial or ampoule (19) having deformable walls with a degree of flexibility and module suitable for facilitating the controlled application of the adhesive through a discharge mouth (20) of said vial or ampoule (19), said discharge mouth (20) being capable of adapting to the cannula (3) and said vial or ampoule (19) being able to be extracted from the device and decoupled from the cannula (3) in order to carry out a replacement once its content has been dispensed.

11. Kit (22) according to claim 10, which comprises a replacement cannula (3) that can be coupled in a detachable manner to said body (2) and two vials or ampoules (19) of adhesive.

12. Kit (22) according to any of claims 10 to 11, wherein said vial or ampoule (19) contains liquid adhesive for medical use for application in the joining of biological human or animal tissues, in the fixing of implants or as a hemostatic.

13. Kit (22) according to any of claims 10 to 12, wherein said device (1) and/or said replacement cannula (3) and/or said vial or ampoule (19) are sterile products, preferably, products sterilised by means of ethylene oxide or gamma radiation.

## Patentansprüche

1. Eine Vorrichtung (1) zum Aufbringen eines medizinischen Flüssighaftmittels aus einer Phiole oder Ampulle (19) mit deformierbaren und flexiblen Wänden bei Offener-Eingriff-Operationen, wobei die Vorrichtung folgende Merkmale aufweist:
einen Körper (2), der mit einem Gehäusehohlraum (8) der Phiole oder Ampulle (19) versehen ist, eine Kanüle (3) zum Führen des Haftmittelabflusses aus der Phiole oder Ampulle (19) und eine Einrichtung (4, 5, 6, 17, 18) zum Betätigen der Wände der Phiole oder Ampulle (19), wobei die Betätigungseinrichtung (4, 5, 6, 17, 18) in der Lage ist, eine Kompressionskraft auf die Wände der Phiole oder Ampulle (19) auszuüben, um das Haftmittel abzugeben, wobei
die Betätigungseinrichtung eine Festspannzange (4) aufweist, die auf einem Träger (5) innerhalb des Körpers (2) befestigt ist, wobei die Arme der Zange (4) ein Paar von Betätigungselementen (6), die einander mit einer relativen Bewegung bezüglich des Körpers (2) gegenüberliegen, wobei der Träger (5) als ein Schwenkelement für die Arme (6) dient und der Körper (2) der Vorrichtung (1) mit einem Paar von Aperturen (7) versehen ist, durch die die Arme (6) der Zange (4) heraustreten, um betätigt zu werden, wobei ein Abschnitt jedes der Arme (6) in Übereinstimmung mit dem Gehäusehohlraum (8) der Phiole oder Ampulle (19) angeordnet ist und jeder der Arme (6) einen Kompressionszahn (17) der Phiole oder Ampulle (19) umfasst, wobei die Kompressionszähne (17) zwei gegenüberliegende Druckpunkte bestimmen, durch die eine Deformation von flexiblen Wänden der Phiole oder Ampulle (19) erreicht wird, um das Haftmittel aus der Phiole oder Ampulle (19) durch die Kanüle (3) zu pumpen, wobei
der Körper (2) der Vorrichtung (1) eine Apertur (11) aufweist, die ausgebildet ist, das Einbringen der Phiole oder Ampulle (19) in den zuvor erwähnten Gehäusehohlraum (8) zu ermöglichen, wobei die Kanüle (3) einen Verbindungsabschnitt (3a) umfasst, der dem Körper (2) zugeordnet ist, wobei der Verbindungsabschnitt (3a) der Kanüle (3) mit einer Einrichtung (12a) zum Koppeln einer Abflussöffnung (20) der Phiole oder Ampulle (19) versehen ist, wobei es möglich ist, die Phiole oder Ampulle (19) von der Kanüle (3) zu entkoppeln und dieselbe durch die zuvor erwähnte Apertur (12) herauszuziehen, um eine Ersetzung auszuführen, sobald die Flüssigkeit der Phiole oder Ampulle (19) abgegeben wurde.

2. Vorrichtung gemäß Anspruch 1, bei der die Apertur (11) für das Einbringen der Phiole oder Ampulle (19) in einem Ende (2a) des Körpers (2) vorgesehen ist, wobei die Kanüle (3) der Vorrichtung einen Verbindungsabschnitt (3a) umfasst, der auf eine abnehmbare Weise mit dem offenen Ende (2a) gekoppelt sein kann, wobei es möglich ist, die Phiole oder Ampulle (19) zusammen mit der Kanüle (3) durch das offene Ende (2a) herauszuziehen, um eine Ersetzung auszuführen.

3. Vorrichtung gemäß Anspruch 1, bei der die innere Kante des Abschnitts jedes der Arme (6) einen der Kompressionszähne (17) und ein Element (18) aufweist, das für die Phiole oder Ampulle (19) als ein Anschlag dient, wobei die Elemente (18), die als ein Anschlag dienen, das Sichern der Phiole oder Ampulle (19) ermöglichen, wenn die Kompressionszähne (17) die Wände der Phiole oder Ampulle (19) deformieren.

4. Vorrichtung gemäß einem der vorhergehenden Ansprüche, bei der der Gehäusehohlraum (8) des Körpers ein Trägerelement (9) für die Basis (19a) der Phiole oder Ampulle (19) und seitliche Führungsschienen (10) aufweist, um die Position der Phiole oder Ampulle (19) zu führen.

5. Vorrichtung gemäß einem der Ansprüche 2 bis 4, bei der der Verbindungsabschnitt (3a) der Kanüle (3) eine Einbringungswand (14) in der Apertur (11) des Endes (2a) des Körpers (2) aufweist, wobei die Einbringungswand (14) und die Wand, die die Apertur (11) des Endes (2a) definiert, eine Einrichtung zur gegenseitigen Eingriffnahme (15, 16a, 16b) für ein sicheres Koppeln umfassen.

6. Vorrichtung gemäß einem der vorhergehenden Ansprüche, bei der die Kopplungseinrichtung der Abflussöffnung (20) der Phiole oder Ampulle (19) einen Hohlraum (12a) aufweist, der proportioniert ist, um ein Einstellen der Abflussöffnung (20) der Phiole oder Ampulle (19) zu ermöglichen.

7. Vorrichtung gemäß einem der vorhergehenden Ansprüche, bei der das freie Ende der Kanüle eine Biegung aufweist, die eine Kurve bildet.

8. Vorrichtung gemäß einem der vorhergehenden Ansprüche, bei der die Spitze der Kanüle (3) ein Segment einer kleineren Größe darstellt, so dass dieselbe abgeschnitten werden kann.

9. Vorrichtung gemäß einem der vorhergehenden Ansprüche, bei der der Körper (2) ein seitliches Fenster (21) aufweist, das in Übereinstimmung mit dem Gehäusehohlraum (8) der Phiole oder Ampulle (19) positioniert ist, wobei das Fenster (21) es ermöglicht, den Haftmittelpegel der Phiole oder Ampulle (19) zu sehen.

10. Eine Medizinisches-Haftmittel-Spender-Ausrüstung (22), die eine Vorrichtung (1) gemäß einem der Ansprüche 1 bis 9 und eine Phiole oder Ampulle (19) aufweist, die ein Flüssighaftmittel zur medizinischen Verwendung enthält, wobei die Phiole oder Ampulle (19) deformierbare Wände mit einem Grad an Flexibilität und ein Modul aufweist, das geeignet ist, ein gesteuertes Aufbringen des Haftmittels durch eine Abflussöffnung (20) der Phiole oder Ampulle (19) zu ermöglichten, wobei die Abflussöffnung (20) sich an die Kanüle (3) anpassen kann, und die Phiole oder Ampulle (19) aus der Vorrichtung herausgezogen werden kann und von der Kanüle (3) entkoppelt werden kann, um eine Ersetzung auszuführen, sobald der Inhalt derselben abgegeben wurde.

11. Eine Ausrüstung (22) gemäß Anspruch 10, die eine Ersatzkanüle (3) aufweist, die auf eine abnehmbare Weise mit dem Körper (2) und zwei Haftmittelphiolen oder-ampullen (19) gekoppelt werden kann.

12. Eine Ausrüstung (22) gemäß einem der Ansprüche 10 bis 11, bei der die Phiole oder Ampulle (19) ein Flüssighaftmittel zur medizinischen Verwendung zum Aufbringen bei dem Verbinden von biologischen menschlichen oder tierischen Geweben, bei dem Befestigen von Implantaten oder als ein Hämostatikum enthält.

13. Eine Ausrüstung (22) gemäß einem der Ansprüche 10 bis 12, bei der die Vorrichtung (1) und/oder die Ersatzkanüle (3) und/oder die Phiole oder Ampulle (19) sterile Produkte sind, vorzugsweise Produkte, die mittels Ethylenoxid oder Gammastrahlung sterilisiert sind.

## Revendications

1. Dispositif (1) pour appliquer un adhésif médical liquide lors d'opérations chirurgicales ouvertes à partir d'une fiole ou ampoule (19) avec des parois déformables ou souples, le dispositif comprenant un corps (2) doté d'une cavité encastrée (8) de ladite fiole ou ampoule (19), une canule (3) pour guider le déversement de l'adhésif de ladite fiole ou ampoule (19) et des moyens (4, 5, 6, 17, 18) pour activer les parois de ladite fiole ou ampoule (19), lesdits moyens d'activation (4, 5, 6, 17, 18) étant capables d'exercer une force de compression sur les parois de ladite fiole ou ampoule (19) pour distribuer ledit adhésif, dans lequel,
- lesdits moyens d'activation comprennent une tenaille de serrage (4) montée sur un support (5) à l'intérieur dudit corps (2), les bras de ladite tenaille (4) configurant une paire d'éléments activateurs (6) opposés l'un à l'autre et en mouvement par rapport audit corps (2), ledit support (5) agissant comme un pivot pour lesdits bras (6) et le corps (2) dudit dispositif (1) étant muni d'une paire d'ouvertures (7) à travers lesquelles les bras (6) de ladite tenaille (4) émergent pour être activés, une partie de chacun desdits bras (6) étant disposée en correspondance avec la cavité encastrée (8) de ladite fiole ou ampoule (19) et chacun desdits bras (6) comprenant une dent de compression (17) de ladite fiole ou ampoule (19), ladite dent de compression (17) établissant deux points de pression opposés à travers lesquels la déformation des parois souples de ladite fiole ou ampoule (19) est réalisée pour pomper ledit adhésif de ladite fiole ou ampoule (19) à travers la canule (3),
dans lequel,
- le corps (2) dudit dispositif (1) comprend une ouverture (11) configurée pour permettre l'insertion de ladite fiole ou ampoule (19) dans la cavité encastrée susmentionnée (8), ladite canule (3) comprenant une partie de jonction (3a) associée audit corps (2), ladite partie de jonction (3a) de la canule (3) étant munie de moyens (12a) de couplage d'une bouche de déversement (20) de ladite fiole ou ampoule (19), le découplage de ladite fiole ou ampoule (19) de ladite canule (3) étant possible, ainsi que son extraction à travers l'ouverture susmentionnée (11) afin de réaliser un remplacement une fois le liquide de ladite fiole ou ampoule (19) distribué.

2. Dispositif selon la revendication 1, dans lequel ladite ouverture (11) pour l'insertion de la fiole ou ampoule (19) est prévue dans l'une des extrémités (2a) dudit corps (2), la canule (3) du dispositif comprenant une partie de jonction (3a) pouvant être couplée de façon détachable à ladite extrémité ouverte (2a), ladite fiole ou ampoule (19) pouvant être extraite à travers ladite extrémité ouverte (2a) conjointement à la canule (3) afin d'effectuer un remplacement.

3. Dispositif selon la revendication 1, dans lequel le bord interne de la partie de chacun desdits bras (6) comprend l'une desdites dents de compression (17) et un élément (18) agissant comme un butoir pour ladite fiole ou ampoule (19), lesdits éléments (18) agissant comme un butoir facilitant la fixation de ladite fiole ou ampoule (19) lorsque lesdites dents de compression (17) déforment les parois de ladite fiole ou ampoule (19).

4. Dispositif selon une quelconque des revendications précédentes, dans lequel la cavité encastrée (8) dudit corps comprend un élément de soutien (9) pour la base (19a) de ladite fiole ou ampoule (19) et des rails de guidage latéraux (10) pour guider la position de ladite fiole ou ampoule (19).

5. Dispositif selon une quelconque des revendications 2 à 4 dans lequel la partie de jonction (3a) de la canule (3) comprend une paroi d'insertion (14) dans l'ouverture (11) de l'extrémité (2a) dudit corps (2), ladite paroi d'insertion (14) et la paroi définissant l'ouverture (11) de ladite extrémité (2a) comprenant des moyens d'engagement mutuel (15, 16a, 16b) pour un couplage sécurisé.

6. Dispositif selon une quelconque des revendications précédentes, dans lequel les moyens de couplage de la bouche de déversement (20) de ladite fiole ou ampoule (19) comprend une cavité (12a) dimensionnée pour permettre l'ajustement de la bouche de déversement (20) de ladite fiole ou ampoule (19).

7. Dispositif selon une quelconque des revendications précédentes, dans lequel l'extrémité libre de la canule comprend une pliure définissant une courbe.

8. Dispositif selon une quelconque des revendications précédentes, dans lequel l'extrémité de la canule (3) présente une section de taille inférieure afin qu'elle puisse être coupée.

9. Dispositif selon une quelconque des revendications précédentes, dans lequel ledit corps (2) comprend une fenêtre latérale (21) placée en correspondance de la cavité encastrée (8) de ladite fiole ou ampoule (19), ladite fenêtre (21) rendant possible la visualisation du niveau de l'adhésif de la fiole ou ampoule (19).

10. Kit de distribution d'adhésif médical (22) comprenant un dispositif (1) selon une quelconque des revendications 1 à 9 et une fiole ou ampoule (19) contenant un liquide adhésif à usage médical, ladite fiole ou ampoule (19) étant munie de parois déformables avec un degré de souplesse et un module destiné à faciliter une application contrôlée de l'adhésif à travers une bouche de déversement (20) de ladite fiole ou ampoule (19), ladite bouche de déversement (20) pouvant être adaptée à la canule (3) et ladite fiole ou ampoule (19) pouvant être extraite du dispositif et découplée de la canule (3) afin d'effectuer un remplacement une fois son contenu ayant été distribué.

11. Kit (22) selon la revendication 10 comprenant une canule de remplacement (3) pouvant être couplée de façon détachable audit corps (2) et deux fioles ou ampoules (19) d'adhésif.

12. Kit (22) selon une quelconque des revendications 10 à 11, dans lequel ladite fiole ou ampoule (19) contient un adhésif liquide à usage médical pour application dans la jointure de tissus biologiques humains ou animaliers, dans la fixation d'implants ou en tant qu'hémostatique.

13. Kit (22) selon une quelconque des revendications 10 à 12, dans lequel ledit dispositif (1) et/ou ladite canule de remplacement (3) et/ou ladite fiole ou ampoule (19) sont des produits stériles, de préférence, des produits stérilisés au moyen d'oxyde d'éthylène ou de radiation gamma.
